# EUROPEAN PATENT APPLICATION

(11) **EP 4 732 855 A1**
(43) Date of publication of application: **29.04.2026**
(21) Application number: 24825773.5
(22) Date of filing: 10.06.2024
(51) Int. Cl.: A61L 9/01, A61L 9/14

(54) **HYDROGEN PEROXIDE DECONTAMINATION SYSTEM**

(30) Priority: 21.06.2023 JP 2023101602
(71) Applicant: Airex Co., Ltd., Nagoya-shi Aichi 453-0015 (JP)
(72) Inventor: KAWASAKI, Koji, Nagoya-shi, Aichi 453-0015 (JP); OGATA, Yoshitaka, Nagoya-shi, Aichi 453-0015 (JP)
(74) Representative: Kraus & Lederer PartGmbB
(86) International application number: PCT/JP2024/021016
(87) International publication number: WO 2024/262368

(57) **Abstract**

The present invention provides a hydrogen peroxide decontamination system capable of accurately controlling the concentration of hydrogen peroxide gas in a room to be decontaminated and the concentration of hydrogen peroxide in a condensed film by accurately identifying the amount and the concentration of a hydrogen peroxide solution supplied to the room to be decontaminated and preparing an environment where a uniform condensed film is formed within the room to be decontaminated.

In a step 1, an air circulating means is stopped while the temperature and the humidity in a working chamber are stable.

In a step 2, while a hydrogen peroxide solution is supplied from a storage tank to a mist converting/supplying means, the weight and the concentration of the supplied hydrogen peroxide solution are calculated over time.

Furthermore, the mist converting/supplying means is operated to convert the hydrogen peroxide solution into a mist for decontamination, and a mist diffusion means is operated to diffuse the mist for decontamination into the working chamber.

In a step 3, the mist diffusion means is maintained in an operated state for a predetermined decontamination duration.

In a step 4, an aeration means is operated at the end of the decontamination duration.

## Description

### TECHNICAL FIELD

The present invention relates to a hydrogen peroxide decontamination system for decontaminating the inside such as a clean room and an isolator device with hydrogen peroxide, and particularly to a hydrogen peroxide decontamination system that essentially confirms the state of gas-liquid equilibrium.

### BACKGROUND ART

In manufacturing settings for pharmaceutical or food products or in the clinical environment such as operating rooms, the indoor working area must inevitably be kept sterile. Particularly in cases where clean rooms as a working chamber for manufacturing pharmaceutical products are decontaminated, advanced decontamination validation needs to be accomplished in accordance with Good Manufacturing Practice (GMP).

In recent years, hydrogen peroxide has widely been used to decontaminate a working chamber such as a sterile room (hereinafter referred to as a "room to be decontaminated"). Advantageously, hydrogen peroxide has a strong sterilization effect, and is inexpensively available and effectively utilized as an environmentally-friendly decontamination gas that is ultimately resolved into oxygen and water.

The conventional and current mainstream decontamination approach is to generate hydrogen peroxide gas by heating and evaporating a hydrogen peroxide solution inside a room to be decontaminated. This approach is called as "flash evaporation decontamination method". In this method, for example, a 30 to 35W/V% hydrogen peroxide solution is fed from the outside to the inside of a room to be decontaminated and heated in a high-temperature evaporation apparatus provided inside the room to be decontaminated to generate hydrogen peroxide gas and water vapor. Thereafter, the air inside the room to be decontaminated is circulated to fill the room with hydrogen peroxide gas. This flash evaporation decontamination method detects the concentration of hydrogen peroxide gas within the room to be decontaminated and defines it as a parameter for decontamination effects.

Meanwhile, the following patent document 1 describes that a decontamination effect by hydrogen peroxide is provided by a condensed film of a hydrogen peroxide solution that condenses on the surface of an object to be decontaminated (a wall surface and an object accommodated inside). Nevertheless, although the flash evaporation decontamination method defines the concentration of hydrogen peroxide gas as a parameter for decontamination effects, a gas concentration meter for hydrogen peroxide has a limited reliability and the concentration of hydrogen peroxide gas in decontamination conditions is considered merely as a reference value rather than a crucial factor. In fact, there are various grades and values of standard of hydrogen peroxide solutions used in decontamination processes, with standards of concentration varying significantly, and the concentration of hydrogen peroxide solutions in stock changes gradually, but constantly over time. In this way, unfortunately, the concentration of hydrogen peroxide solutions supplied into a room to be decontaminated for decontamination operations fails to be accurately controlled, and when the concentration of the hydrogen peroxide solutions changes, the relationship between the concentration of hydrogen peroxide gas inside the room to be decontaminated and the concentration of hydrogen peroxide in a condensed film condensed in the room is insufficiently identified to perform decontamination operations.

Another problem with the flash evaporation decontamination method is associated with circulation of air inside a room to be decontaminated with a circulating fan, leading to unsuccessful formation of a uniform condensed film throughout the room due to differences in air volume and airspeed, depending on parts in the room. A further drawback is that no uniform condensed film can be formed throughout the room from changes in indoor temperature distribution because a high-temperature evaporation apparatus evaporates hydrogen peroxide solutions. Thus, in order to eliminate non-uniform decontamination effects of a room to be decontaminated according to the flash evaporation decontamination method, it is necessary to set conditions for forming an excessive condensed film by increasing the volume of a hydrogen peroxide solution inputted and the volume of the resulting hydrogen peroxide gas supplied.

### CITATION LIST

### PATENT LITERATURE

Patent Document 1: JP-A-61-004543

### SUMMARY OF INVENTION

### TECHNICAL PROBLEM

In fact, when excessive amounts of hydrogen peroxide gas are supplied to a room to be decontaminated, some indoor parts are prone to excessive condensation and each manufacturing facility, precision measuring equipment placed inside a room to be decontaminated and wall surfaces of rooms to be decontaminated are subjected to corrosion by a condensed film by the resulting high concentration of hydrogen peroxide. After a decontamination work using hydrogen peroxide, aeration is performed with clean air to remove the residual hydrogen peroxide gas and condensed film of a hydrogen peroxide solution inside the room to be decontaminated. However, the supply of such an excessive amount of hydrogen peroxide gas is problematic due to longer duration required in the aeration operation for removing a high concentration of condensed film of a hydrogen peroxide solution generated on wall surfaces and other portions of the room to be decontaminated.

Therefore, objectives of the flash evaporation decontamination method, including reduction in hydrogen peroxide solution consumption, shortening of decontamination cycle time, and reduction in concentration of residual gas after decontamination, can desirably be accomplished by a decontamination method by focusing on a condensed film of hydrogen peroxide solution. To accomplish these objectives, a method for directly supplying mists of a hydrogen peroxide solution, in place of hydrogen peroxide gas, to the inside of a room to be decontaminated has been implemented.

This approach is called as "mist introducing decontamination method". This approach allows a two-fluid nozzle or other tools to convert a hydrogen peroxide solution at normal temperature and compressed air into hydrogen peroxide solution mists, which will be supplied into the inside of a room to be decontaminated. However, this approach fails to uniformly decontaminate a room to be decontaminated, because non-uniform condensation can occur at some parts in the room due to mist diffusion with highspeed ejection of compressed air. Accordingly, in order for this approach to acquire decontamination effects throughout the room, excessive amounts of hydrogen peroxide solution mist must inevitably be supplied.

In addition, this approach similarly detects the concentration of hydrogen peroxide gas within a room to be decontaminated as a parameter for decontamination effects, but fails to accurately control the concentration of a hydrogen peroxide solution supplied to the room to be decontaminated, and when the concentration of a hydrogen peroxide solution fed changes, the relationship between the concentration of hydrogen peroxide gas inside the room to be decontaminated and the concentration of hydrogen peroxide in a condensed film condensed in the room is insufficiently identified to perform decontamination operations.

Thus, the present invention was made in view of the situation to solve the problems, and has an object to provide a hydrogen peroxide decontamination system capable of accurately controlling the concentration of hydrogen peroxide gas in a room to be decontaminated and the concentration of hydrogen peroxide in a condensed film by accurately identifying the amount and the concentration of a hydrogen peroxide solution supplied to the room to be decontaminated and preparing an environment where a uniform condensed film is formed within the room to be decontaminated.

### SOLUTION TO PROBLEM

To solve the aforementioned problem, inventors of the present invention have carried out an extended investigation to find that it is possible to confirm the state of gas-liquid equilibrium of a hydrogen peroxide solution by accurately confirming the concentration of a supplied hydrogen peroxide used in decontamination processes, maintaining the inside of a room to be decontaminated in a no-wind state, and preparing an environment for decontamination where no material balance or heat balance occurs during decontamination processes. Based on that technique, the present invention was accomplished.

Specifically, a hydrogen peroxide decontamination system according to the present invention is, according to description in claim 1,
a hydrogen peroxide decontamination system (100) for decontaminating the inside of a working chamber, including
a working chamber (C3), an air circulating means (20), a hydrogen peroxide solution supplying means (30), a mist converting/supplying means (40), a mist diffusion means (50), and an aeration means (60), characterized in that
the working chamber includes an air inlet (13) and an air outlet (14) for switching between a communicating state between the inside of the working chamber and the external environment and a sealed state inside the working chamber, and a temperature measuring device (15) and a humidity measuring device (16) to measure the temperature and humidity in the working chamber over time,
the air circulating means includes an air circulating fan (21) and a high-performance air filter (22) to circulate clean air in the working chamber,
the hydrogen peroxide solution supplying means includes a storage tank (31), a supply pump (32), a supply pipe (33), a metering device (34) and a concentration measuring device (35) to supply a predetermined amount of hydrogen peroxide solution to the mist converting/supplying means,
the mist converting/supplying means converts the hydrogen peroxide solution supplied from the hydrogen peroxide solution supplying means into a mist for decontamination to supply the mist for decontamination to the inside of the working chamber,
the mist diffusion means includes a vibrating board (51) disposed in the working chamber, and the vibrating board is subjected to ultrasonic vibration to generate sound flows from board surfaces thereof by ultrasound and diffuse the mist for decontamination supplied to the working chamber to the inside of the working chamber, and
the aeration means includes an air supply device (61), an air exhaust device (62) and a hydrogen peroxide decomposition apparatus (63) to replace the air introduced via the air inlet from the external environment of the working chamber with clean air, introduce the clean air into the working chamber, and discharge the clean air toward the external environment via the hydrogen peroxide decomposition apparatus and the air outlet together with the hydrogen peroxide residual in the working chamber after decontamination,
the system being operated by the following 5 steps, characterized in that
in a step 1, the air inlet and the air outlet are closed, with the air circulating means operated, to maintain the working chamber in a sealed state and stop the air circulating means while the temperature and humidity in the working chamber are stable,
in a step 2, while the hydrogen peroxide solution supplying means is operated to supply a hydrogen peroxide solution from the storage tank to the mist converting/supplying means via the supply pump and the supply pipe, the weight and concentration of the hydrogen peroxide solution supplied to the mist converting/supplying means as a hydrogen peroxide solution are calculated over time from the amount and the concentration of the hydrogen peroxide solution supplied by the metering device and the concentration measuring device, and
while the mist converting/supplying means is operated to convert the total amount of the hydrogen peroxide solution supplied from the hydrogen peroxide solution supplying means into a mist for decontamination and supply the mist to the working chamber, the mist diffusion means is operated to diffuse the mist for decontamination to the inside of the working chamber,
in a step 3, at the end of supply of a mist for decontamination to the working chamber by the mist converting/supplying means, the mist converting/supplying means is stopped to maintain the mist diffusion means in an operated state for a predetermined decontamination duration,
in a step 4, at the end of the predetermined decontamination duration by the mist diffusion means, the mist diffusion means is stopped to open the air inlet and the air outlet, and operate the air circulating means and the aeration means, and
in a step 5, at the end of a predetermined aeration duration, the air circulating means and the aeration means are stopped and the air inlet and the air outlet are closed to maintain the working chamber in a sealed state,
characterized in that
based upon the temperature and humidity in the working chamber measured over time by the temperature measuring device and the humidity measuring device and the weight and concentration of the hydrogen peroxide solution supplied to the working chamber measured over time by the metering device and the concentration measuring device,
the concentration of hydrogen peroxide gas in the working chamber is continuously calculated, using gas-liquid equilibrium of hydrogen peroxide - water.

Moreover, the present invention is, according to description in claim 2, the hydrogen peroxide decontamination system according to claim 1, characterized in that
in the step 3, the sealed state inside the working chamber is maintained, and the working chamber is decontaminated, with no material and/or heat flowing from the external environment into the working chamber.

Furthermore, the present invention is, according to claim 3, the hydrogen peroxide decontamination system according to claim 1 or 2, characterized in that
the mist diffusion means includes one or more vibrating boards (51), characterized in that
the vibrating board includes a base (52) and a plurality of transmitters (53), and the plurality of transmitters is arranged on a plain surface of the base so as to be uniform in transmission directions, and the transmitters are operated in the same phase, and consequently
a sound flow is generated by a significantly directional ultrasound from a board surface of the vibrating board in the vertical direction by mutually amplifying ultrasounds of the plurality of transmitters in the front direction and mutually canceling out the ultrasounds of the plurality of transmitters in the lateral direction.

### ADVANTAGEOUS EFFECTS OF INVENTION

According to the above configuration, a hydrogen peroxide decontamination system according to the present invention includes a working chamber, an air circulating means, a hydrogen peroxide solution supplying means, a mist converting/supplying means, a mist diffusion means and an aeration means.

The working chamber includes an air inlet and an air outlet for switching between a communicating state between the inside of the working chamber and the external environment and a sealed state inside the working chamber, and a temperature measuring device and a humidity measuring device to measure the temperature and humidity in the working chamber. The air circulating means includes an air circulating fan and a high-performance air filter to circulate clean air in the working chamber. The hydrogen peroxide solution supplying means includes a storage tank, a supply pump, a supply pipe, a metering device and a concentration measuring device to supply a predetermined amount of hydrogen peroxide solution to the mist converting/supplying means.

The mist converting/supplying means converts the hydrogen peroxide solution supplied from the hydrogen peroxide solution supplying means into a mist for decontamination to supply the mist for decontamination to the inside of the working chamber. The mist diffusion means includes a vibrating board disposed in the working chamber, and the vibrating board is subjected to ultrasonic vibration to generate sound flows from board surfaces thereof by ultrasound and diffuse the mist for decontamination supplied to the working chamber to the inside of the working chamber. The aeration means includes an air supply device, an air exhaust device and a hydrogen peroxide decomposition apparatus to replace the air introduced via the air inlet from the external environment of the working chamber with clean air, introduce the clean air into the working chamber, and discharge the clean air toward the external environment via the hydrogen peroxide decomposition apparatus and the air outlet together with the hydrogen peroxide residual in the working chamber after decontamination.

With these configurations, in a step 1, the air inlet and the air outlet are closed, with the air circulating means operated, to maintain the working chamber in a sealed state and stop the air circulating means while the temperature and humidity in the working chamber are stable.

Next, in a step 2, while the hydrogen peroxide solution supplying means is operated to supply a hydrogen peroxide solution from the storage tank to the mist converting/supplying means via the supply pump and the supply pipe, the weight and concentration of the hydrogen peroxide solution supplied to the mist converting/supplying means as a hydrogen peroxide solution are calculated over time from the amount and the concentration of a hydrogen peroxide solution supplied by the metering device and the concentration measuring device. Furthermore, while the mist converting/supplying means is operated to convert the total amount of the hydrogen peroxide solution supplied from the hydrogen peroxide solution supplying means into a mist for decontamination and supply the mist to the inside of the working chamber, the mist diffusion means is operated to diffuse the mist for decontamination to the inside of the working chamber.

Next, in a step 3, at the end of supply of a mist for decontamination to the working chamber by the mist converting/supplying means, the mist converting/supplying means is stopped to maintain the mist diffusion means in an operated state for a predetermined decontamination duration. Next, in a step 4, at the end of the predetermined decontamination duration by the mist diffusion means, the mist diffusion means is stopped to open the air inlet and the air outlet, and operate the air circulating means and the aeration means.

Finally, in a step 5, at the end of a predetermined aeration duration, the air circulating means and the aeration means are stopped to close the air inlet and the air outlet and maintain the working chamber in a sealed state. In this state, the hydrogen peroxide decontamination system is finalized. According to the above configuration, based upon the temperature and humidity in the working chamber measured over time by the temperature measuring device and the humidity measuring device and the weight and concentration of a hydrogen peroxide solution supplied to the working chamber measured over time by the metering device and the concentration measuring device, the concentration of hydrogen peroxide gas in the working chamber can continuously be calculated, using gas-liquid equilibrium of hydrogen peroxide - water.

According to the above configuration, based upon the temperature and humidity in the working chamber measured over time by the temperature measuring device and the humidity measuring device and the weight and concentration of the hydrogen peroxide solution supplied to the working chamber measured over time by the metering device and the concentration measuring device, the concentration of hydrogen peroxide gas in the working chamber is continuously calculated, using gas-liquid equilibrium of hydrogen peroxide - water.

Thus, the present invention can provide a hydrogen peroxide decontamination system capable of accurately controlling the concentration of hydrogen peroxide gas in a room to be decontaminated and the concentration of hydrogen peroxide in a condensed film by accurately identifying the amount and the concentration of a hydrogen peroxide solution supplied to the room to be decontaminated and preparing an environment where a uniform condensed film is formed within the room to be decontaminated.

Also, according to the above configuration, in the step 3, the sealed state inside the working chamber is maintained, and the working chamber is decontaminated, with no material and/or heat flowing from the external environment into the working chamber.

According to the above configuration, the mist diffusion means includes one or more vibrating boards, and each of the vibrating boards includes a base and a plurality of transmitters, and the plurality of transmitters is arranged on a plain surface of the base so as to be uniform in transmission directions, and the transmitters are operated in the same phase. Accordingly, a sound flow can be generated by a significantly directional ultrasound from a board surface of the vibrating board in the vertical direction by mutually amplifying ultrasounds of the plurality of transmitters in the front direction and mutually canceling out the ultrasounds of the plurality of transmitters in the lateral direction. Thus, the above operational advantage can more specifically be provided.

### BRIEF DESCRIPTION OF DRAWINGS

FIG. 1 is a schematic side cross-sectional view illustrating the inside of a hydrogen peroxide decontamination system according to the present invention depicted as an isolator;
FIG. 2(A) is a front view of a mist converting/supplying device according to the present embodiment viewed from the inside of a chamber and FIG. 2(B) is a cross-sectional side view of the mist converting/supplying device; and
FIG. 3 is a schematic perspective view illustrating a plurality of ultrasonic speakers arranged in a speaker base in a vibrating board included in a mist diffusion device.

### DESCRIPTION OF EMBODIMENTS

In the present invention, "mist" is broadly interpreted as the state of a liquid droplet of a hydrogen peroxide solution refined and floating in the air, the state of a hydrogen peroxide gas and a liquid droplet of a hydrogen peroxide solution in mixture, the state of the hydrogen peroxide solution to repeat the change in phase between condensation and evaporation of a gas and a droplet, and the like. Accordingly, the mist according to the present invention is categorized into a "mist" (the size may be defined as 10 µm or less) or a "fog" (the size may be defined as 5 µm or less), and a mist having a larger particle size.

In the present invention, ultrasonic vibration of a mist diffusion means converts even a mist, a fog and a liquid droplet sized 3 to 10 µm or more into equalized ultrafine particles 3 µm or less to repeat the change in gas-liquid phase between a hydrogen peroxide gas in the room, a condensed film and a mist and provide high-level decontamination effects.

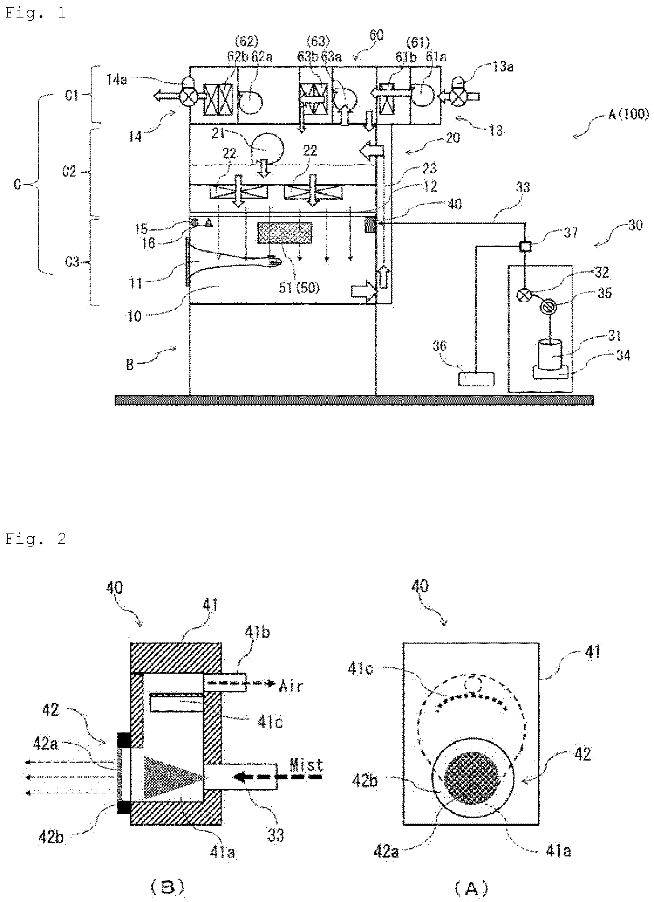

A hydrogen peroxide decontamination system 100 according to the present invention will be described in detail with reference to an embodiment. The present invention is not restricted to the following embodiment only. An embodiment will be described by illustrating an isolator. FIG. 1 is a schematic side cross-sectional view illustrating the inside of an isolator of the embodiment.

In FIG. 1, an isolator device A is composed of a mount B placed on the floor and an isolator body C loaded onto the mount B. The mount B is covered with a wall material formed of a stainless metal plate in circumference, inside of which electric equipment and machines (not shown) are accommodated. The isolator body C is divided into an upper air supply and exhaust chamber C1, an air circulating and driving chamber C2 located thereunder, and a working chamber C3 located thereunder.

Each of the isolator body C (C1, C2, C3) includes, as each component, a chamber 10, an air circulating device 20, a hydrogen peroxide solution supplying device 30, a mist converting/supplying device 40, a mist diffusion device 50, and an aeration device 60.

The working chamber C3 is disposed on the mount B, surrounded and faced by 4 side wall portions, connected to the air circulating and driving chamber C2 via a rectifying screen 12 provided at a ceiling portion thereof, and a work glove 11 is provided on some wall surfaces. The working chamber C3 constitutes the chamber 10 for an operator outside the isolator device A to perform a sterile operation inside the isolator device A via the work glove 11.

The chamber 10 switches between a communicating state with the external environment and a sealed state inside the chamber, with the upper air supply and exhaust chamber C1 including an air inlet 13 and an air supply valve 13a, and an air outlet 14 and an air exhaust valve 14a communicating with the external environment via the air circulating and driving chamber C2. The chamber 10 includes a temperature measuring device 15 and a humidity measuring device 16 to measure the temperature and humidity therein over time. The chamber 10 may be provided with a hydrogen peroxide gas concentration meter and a pressure gauge therein.

The air circulating device 20 is provided with an air circulating fan 21, a HEPA filter 22 and a circulation path 23 in the air circulating and driving chamber C2, and circulates clean air inside the chamber 10 between the air circulating and driving chamber C2.

The hydrogen peroxide solution supplying device 30 is placed outside the isolator body C. The hydrogen peroxide solution supplying device 30 includes a storage tank 31, a supply pump 32, a supply pipe 33, a metering device 34 and a concentration measuring device 35. The supply pipe 33 allows the storage tank 31 and the mist converting/supplying device 40 to communicate with each other, to which pathway the supply pump 32 and the concentration measuring device 35 are connected. The supply pump 32 is operated to supply a hydrogen peroxide solution from the storage tank 31 to the mist converting/supplying device 40 and measure the amount and the concentration of the hydrogen peroxide solution over time. In this embodiment, a two-fluid spray nozzle 37 converting a hydrogen peroxide solution into mists with a compressed air supply device 36 is provided in a pathway of the supply pipe 33 to supply a resulting hydrogen peroxide solution mist (primary mist) to the mist converting/supplying device 40.

The mist converting/supplying device 40 is placed at a region where an upper wall surface and a side wall surface of the chamber 10 intersect. Only a mist discharge port may be provided on an inner wall surface of the chamber 10, and the device body may be provided on an outer wall surface of the chamber 10. The mist converting/supplying device 40 converts a hydrogen peroxide solution supplied from the hydrogen peroxide solution supplying device 30 (a primary mist in this embodiment) into a fine hydrogen peroxide solution mist (a secondary mist) to supply the mist to the inside of the chamber 10.

FIG. 2(A) is a front view of a mist converting/supplying device according to the present invention viewed from the inside of the chamber and FIG. 2(B) is a cross-sectional side view of the mist converting/supplying device. In FIG. 2, the mist converting/supplying device 40 is composed of a mist receiving container 41 and an ultrasonic atomizer 42.

The mist receiving container 41 constitutes a space with a front internal portion having a semi-spindle-shaped cross section, and the ultrasonic atomizer 42 is attached to a front lower end portion with a semi-spindle-shaped focusing width. The lower end portion of the internal space is provided with a focusing width to serve as a liquid pool 41a for a small amount of decontamination liquid separated into gas and liquid. Also, an end of the supply pipe 33 communicates with a rear lower end portion of the mist receiving container 41 (at a position opposite the ultrasonic atomizer 42) toward the inside of the mist receiving container 41. An air vent 41b opens at an upper end portion inside a rear surface of the mist receiving container 41. A baffle plate 41c is provided between the end of the supply pipe 33 in the center inside the mist receiving container 41 and the air vent 41b.

The ultrasonic atomizer 42 is composed of a substantially annular disk-shaped perforated vibration plate 42a provided with a plurality of micropores (not shown) atomizing the hydrogen peroxide solution subjected to gas-liquid separation, the micropores passing through the perforated vibration plate between the front surface and the back surface thereof, a piezoelectric vibrator 42b formed of a substantially annular disk in which the perforated vibration plate 42a is subjected to film vibration, and a control unit (not shown) controlling the vibration of the piezoelectric vibrator 42b. The perforated vibration plate 42a is affixed to the piezoelectric vibrator 42b so as to cover an internal hole portion of the piezoelectric vibrator 42b.

Also, the perforated vibration plate 42a is attached such that the front surface faces the inside of the chamber 10 and the rear surface faces the inside of the mist receiving container 41, and a plurality of micropores of the perforated vibration plate 42a passes through the inside of the room to be decontaminated and the inside of the mist receiving container 41. In FIG. 2, the perforated vibration plate 42a is disposed so as to discharge a hydrogen peroxide mist horizontally from the front surface of the perforated vibration plate 42a, but the configuration is not restricted thereto, and the hydrogen peroxide mist may be discharged downward or upward, depending on the position of the plate disposed.

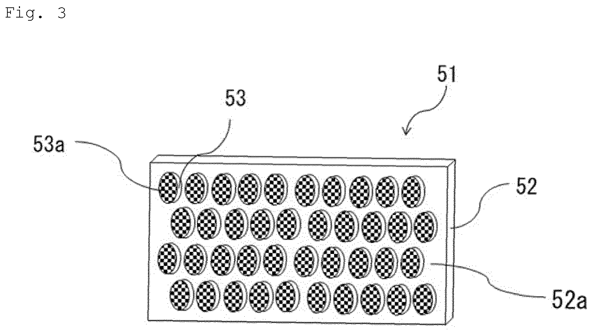

The mist diffusion device 50 includes a vibrating board 51 disposed on an upper side wall surface of the chamber 10 (an upper back wall surface viewed from the front in FIG. 1). The mist diffusion device 50 subjects the vibrating board 51 to ultrasonic vibration to generate sound flows from board surfaces thereof by ultrasound and diffuse a hydrogen peroxide solution mist supplied inside the chamber 10 to the inside of the chamber 10. In this embodiment, one vibrating board 51 is used, but two or more vibrating boards 51 may be used. FIG. 3 is a schematic perspective view illustrating a plurality of ultrasonic speakers arranged in a speaker base in a vibrating board included in a mist diffusion device.

In FIG. 3, the vibrating board 51 includes a base and a plurality of transmitters. In this embodiment, the base used is a speaker base 52, and the transmitter used is an ultrasonic speaker 53. In this embodiment, 40 ultrasonic speakers 53 are arranged on a plain surface 52a of the speaker base 52 so as to be uniform in transmission direction of a vibrating surface 53a (leftward as seen from the front shown). The number of ultrasonic speakers is not particularly restricted.

In this embodiment, the ultrasonic speaker 53 used is an ultra directional ultrasonic speaker. Specifically, an ultrasonic speaker (DC12V, 50mA) of frequency modulation system for transmitting an ultrasound whose frequency is around 40 KHz is used. The type, size, structure and output of the ultrasonic speaker are not particularly restricted. In the present invention, the vibrating board included in the mist diffusion device 50 is not restricted to an ultrasonic speaker, and the ultrasonic generation mechanism, frequency range and output are not particularly restricted.

In this embodiment, a plurality of (40) ultrasonic speakers 53 are arranged so as to be uniform in transmission direction of the vibrating surface 53a, and the transmitters are operated in the same phase to mutually amplify ultrasounds from the plurality of ultrasonic speakers 53 in the front direction and mutually cancel out ultrasounds from the plurality of ultrasonic speakers 53 in the lateral direction. Consequently, the ultrasonic speakers 53 arranged on the speaker base 52 are subjected to ultrasonic vibration to generate a significantly directional sound flow traveling in the air from each of the vibrating surfaces 53a in the vertical direction. The frequency and output of the ultrasonic speakers 53 are controlled by a control unit (not shown) to achieve efficient decontamination operations.

An aeration device 60 communicates with the inside of the chamber 10 via the air circulating and driving chamber C2, with the upper air supply and exhaust chamber C1 including an air supply device 61, an air exhaust device 62 and a hydrogen peroxide decomposition apparatus 63. The air supply device 61 includes an air supply fan 61a and a HEPA filter 61b, the air exhaust device 62 includes an air exhaust fan 62a and a HEPA filter 62b, and the hydrogen peroxide decomposition apparatus 63 includes a hydrogen peroxide decomposition fan 63a and a hydrogen peroxide decomposition filter 63b.

The air supply fan 61a replaces the air introduced from the external environment via the air inlet 12 with clean air, using the HEPA filter 61b, to introduce the air into the chamber 10 via the air circulating fan 21 and the HEPA filter 22 of the air circulating and driving chamber C2. The air introduced into the chamber 10 is introduced together with the hydrogen peroxide residual inside the chamber 10 into the hydrogen peroxide decomposition apparatus 63 via the circulation pathway 23 and the air circulating and driving chamber C2 to decompose the hydrogen peroxide with the hydrogen peroxide decomposition filter 63b. The air exhaust fan 62a discharges the air with decomposed hydrogen peroxide toward the external environment via the HEPA filter 62b and the air outlet 13.

Next, by illustrating the isolator device A with the above configurations, a hydrogen peroxide decontamination system 100 according to the present invention will be described with reference to each operational step. In this embodiment, the hydrogen peroxide decontamination system 100 can be described with 5 operational steps 1 to 5. Additionally, decontamination conditions are predetermined in preliminary experiments and discussions considering the volume of a room to be decontaminated and contents, including an amount of decontamination solution supplied and a supply duration (an amount supplied per unit time), a decontamination duration and an aeration duration.

### <<Step 1>>

A step 1 starts with closing of the air supply valve 13a of the air inlet 13 and the air exhaust valve 14a of the air outlet 14 in the upper air supply and exhaust chamber C1 to maintain the inside of the chamber 10 in a sealed state. Next, the air circulating fan 21 of the air circulating device 20 is operated to circulate the air from the air circulating and driving chamber C2, through the chamber 10 and into the circulation pathway 23. At this time, the chamber 10 includes the temperature measuring device 15 and the humidity measuring device 16 therein to measure the temperature and the humidity inside the chamber 10 over time.

In this embodiment, a record control unit (not shown) is included in the chamber 10 to constantly monitor and record the temperature and the humidity. The chamber 10 may be provided therein with a temperature control unit and a humidity control unit in addition to the temperature measuring device 15 and the humidity measuring device 16 to adjust the temperature and the humidity to a predetermined temperature and humidity. Next, while the temperature and the humidity inside the chamber 10 are stable, the air circulating fan 21 of the air circulating device 20 is stopped. Herein, the inside of the chamber 10 is closed, with no material or heat coming from the external environment.

In this embodiment, the decontamination start temperature and humidity are not particularly required to be determined. In a hydrogen peroxide decontamination system according to the present invention, the inside of the chamber 10 is maintained in a closed system, and a material coming into the system is a hydrogen peroxide solution at normal temperature, with no heat resource heating the material. Thus, temperature changes before and after decontamination operations are extremely small in principle.

### <<Step 2>>

A step 2 is to input hydrogen peroxide into the chamber 10 in decontamination operations. In the step 2, the inside of the chamber 10 is maintained in a sealed state, and the air circulating fan 21 of the air circulating device 20 is stopped to operate the supply pump 32 and the compressed air supply device 36 of the hydrogen peroxide solution supplying device 30. In the meantime, the mist diffusion device 50 is operated, as well as the mist converting/supplying device 40.
Herein, the inside of the chamber 10 is closed, with a material (a hydrogen peroxide solution) coming from the external environment. However, in this embodiment, no heat comes into the chamber due to no heating and vaporizing of a hydrogen peroxide solution.

The supply pump 32 and the compressed air supply device 36 of the hydrogen peroxide solution supplying device 30 are operated to supply a hydrogen peroxide solution (a primary mist) to the operated mist converting/supplying device 40. Accordingly, a refined hydrogen peroxide solution mist (a secondary mist) is discharged toward the inside of the chamber 10, and the hydrogen peroxide solution mist (the secondary mist) is further refined by the operated mist diffusion device 50 and diffused to the inside of the chamber 10.

Herein, the air circulating device 20 is not operated, with no air flowing into the chamber 10. Accordingly, a uniform condensed film can be formed throughout the room due to no differences in air volume and airspeed depending on parts in the room. Further, ultrasonic vibration of the mist diffusion device 50 converts the hydrogen peroxide solution mist (the secondary mist) discharged toward the inside of the chamber 10 into equalized ultrafine particles 3µm or less and repeats gas-liquid phase change between the hydrogen peroxide gas inside the chamber 10 and the condensed film for mist diffusion.

In the step 2, upon supply of a hydrogen peroxide solution by the hydrogen peroxide solution supplying device 30 after activation, the weight of a hydrogen peroxide solution supplied to the mist converting/supplying device 40 by the metering device 34 is precisely measured over time and recorded on a record control unit. At the same time, the concentration of a hydrogen peroxide solution supplied to the mist converting/supplying device 40 by the concentration measuring device 35 provided in the pathway of the supply pipe 33 is precisely measured over time and recorded on the record control unit.

This process is achieved for precisely calculating the concentration of a hydrogen peroxide solution that is variable during storage. The hydrogen peroxide solution is gradually decomposed while it is reserved in the storage tank 31. If concentration changes are not taken into account, the relationship between the amount of hydrogen peroxide supplied to the inside of the chamber 10 and the amount of water is imbalanced, thereby failing to construct a decontamination system for achieving a uniform decontamination by reducing the hydrogen peroxide consumption.

Thus, in the present invention, the weight and concentration (incl. their changes) of the hydrogen peroxide solution supplied to the inside of the chamber 10 are accumulated per unit time. Accordingly, a precise amount of hydrogen peroxide solution supplied to the mist converting/supplying device 40, or the amount of supplied hydrogen peroxide (pure hydrogen peroxide) and the amount of water (pure water) supplied upon supply of a hydrogen peroxide solution can precisely be calculated and recorded as the supply duration increases.

The hydrogen peroxide solution (the primary mist) supplied to the mist converting/supplying device 40 is sequentially converted into a hydrogen peroxide solution mist (a secondary mist) and then supplied to the inside of the chamber 10. The hydrogen peroxide solution mist supplied into the chamber 10 is converted into equalized ultrafine particles 3µm or less by the mist diffusion device 50 to repeat the change in phase between condensation and evaporation for mist diffusion into the chamber 10 and form a uniform condensed film inside the chamber 10.

### <<Step 3>>

A step 3 is to complete decontamination operations, with no flow of a material or heat coming from the external environment in the decontamination operations. In the step 3, the supply of a predetermined amount of hydrogen peroxide solution by the hydrogen peroxide solution supplying device 30 is completed, and the supplied hydrogen peroxide solution is converted into a hydrogen peroxide solution mist (a secondary mist) by the mist converting/supplying device 40 to stop the mist converting/supplying means 40 at the end of the mist supply to the inside of the chamber (10). At this time, the operation of the mist diffusion device 50 continues for a predetermined decontamination duration.

Herein, the inside of the chamber 10 is maintained in a closed system, and the hydrogen peroxide solution supplying device 30 and the mist converting/supplying device 40 are stopped, with only the mist diffusion device 50 operated. In this state, there is no air flow into the chamber 10, and only sound flows by ultrasonic vibration are diffused and reflected. In this state, a uniform condensed film can be formed throughout the room due to no differences in air volume and airspeed depending on parts in the room. Therefore, with no flow of a material or heat coming from the external environment, decontamination processes are performed by confirming the state of gas-liquid equilibrium until stable decontamination is completed.

Herein, how the gas-liquid equilibrium is confirmed in the step 3 will be described. Upon completion of the step 1, the inside of the chamber 10 is maintained in a closed system, and the amount of the water present can be calculated from the corresponding temperature and humidity and the volume of the chamber 10. Upon completion of the step 2, a precise amount of hydrogen peroxide solution supplied to the inside of the chamber 10 (an amount of hydrogen peroxide and an amount of water as its solvent) can be determined by calculation and recording.

Thus, the state of gas-liquid equilibrium of hydrogen peroxide - water inside the chamber 10 can be calculated over time and confirmed. Herein, according to gas-liquid equilibrium phase diagram of hydrogen peroxide and water from the amount of hydrogen peroxide, amount of water, the temperature, the humidity, and pressure conditions inside the chamber 10, the concentration of hydrogen peroxide gas in the system can be calculated. Consequently, since the concentration of hydrogen peroxide gas in the system can precisely be identified, as opposed to conventionally insufficiently controlled with hydrogen peroxide gas concentration meters, the state of more proper decontamination effects can be confirmed.

### <<Step 4>>

A step 4 is to start an aeration operation upon completion of the decontamination operations. In the step 4, at the end of the decontamination duration predetermined in the above step 3, the operation of the mist diffusion device 50 is stopped. Next, the air supply valve 13a of the air inlet 13 and the air exhaust valve 14a of the air outlet 14 in the upper air supply and exhaust chamber C1 are opened to operate the aeration device 60, as well as the air circulating fan 21 of the air circulating device 20. The operational time of the aeration device 60 is determined under predetermined conditions.

### <<Step 5>>

A step 5 is to start a sterile operation inside the chamber 10 upon completion of the aeration operation. In the step 5, at the end of the aeration duration predetermined in the above step 4, the air circulating fan 21 of the air circulating device 20 and the aeration device 60 are stopped. Next, the air supply valve 13a of the air inlet 13 and the air exhaust valve 14a of the air outlet 14 in the upper air supply and exhaust chamber C1 are closed to maintain the inside of the chamber 10 in a sealed state.

In this way, the hydrogen peroxide decontamination system 100 of the isolator device A is finalized. Thus, this embodiment can provide a hydrogen peroxide decontamination system capable of accurately controlling the concentration of hydrogen peroxide gas in a room to be decontaminated and the concentration of hydrogen peroxide in a condensed film by accurately identifying the amount and the concentration of a hydrogen peroxide solution supplied to the room to be decontaminated and preparing an environment where a uniform condensed film is formed within the room to be decontaminated.

### REFERENCE SIGNS LIST

10...Chamber, 11...Work glove, 13...Air inlet, 13a...Air supply valve,
12...Rectifying screen, 14...Air outlet, 14a...Air exhaust valve,
15...Temperature measuring device, 16...Humidity measuring device,
20...Air circulating device, 21...Air circulating fan,
22...HEPA filter, 23...Circulation pathway,
30...Hydrogen peroxide solution supplying device, 31...Storage tank, 32...Supply pump,
33...Supply pipe, 34...Metering device, 35...Concentration measuring device,
36...Compressed air supply device, 37...Two-fluid spray nozzle,
40...Mist converting/supplying device, 41...Mist receiving container, 41a...Liquid pool, 41b...Air vent, 41c...Baffle plate,
42...Ultrasonic atomizer, 42a...Perforated vibration plate, 42b...Piezoelectric vibrator,
50...Mist diffusion device, 51...Vibrating board, 52...Speaker base, 52a...Plain surface of base,
53...Ultrasonic speaker, 53a...Vibrating surface,
60...Aeration device, 61...Air supply device, 61a...Air supply fan,
61b...HEPA filter, 62...Air exhaust device, 62a...Air exhaust fan,
62b...HEPA filter, 63...Hydrogen peroxide decomposition apparatus,
63a...Hydrogen peroxide decomposition fan, 63b...Hydrogen peroxide decomposition filter,
100... Hydrogen peroxide Decontamination system,
A...Isolator, B...Mount, C...Isolator body, C1...Upper air supply and exhaust chamber,
C2...Air circulating and driving chamber, C3...Working chamber.

## Claims

1. A hydrogen peroxide decontamination system for decontaminating the inside of a working chamber, comprising
a working chamber, an air circulating means, a hydrogen peroxide solution supplying means, a mist converting/supplying means, a mist diffusion means and an aeration means, wherein
the working chamber includes an air inlet and an air outlet for switching between a communicating state between the inside of the working chamber and the external environment and a sealed state inside the working chamber, and a temperature measuring device and a humidity measuring device to measure the temperature and humidity in the working chamber over time,
the air circulating means includes an air circulating fan and a high-performance air filter to circulate clean air in the working chamber,
the hydrogen peroxide solution supplying means includes a storage tank, a supply pump, a supply pipe, a metering device and a concentration measuring device to supply a predetermined amount of hydrogen peroxide solution to the mist converting/supplying means,
the mist converting/supplying means converts the hydrogen peroxide solution supplied from the hydrogen peroxide solution supplying means into a mist for decontamination to supply the mist for decontamination to the inside of the working chamber,
the mist diffusion means includes a vibrating board disposed in the working chamber, and the vibrating board is subjected to ultrasonic vibration to generate sound flows from board surfaces thereof by ultrasound and diffuse the mist for decontamination supplied to the working chamber into the working chamber, and
the aeration means includes an air supply device, an air exhaust device and a hydrogen peroxide decomposition apparatus to replace the air introduced via the air inlet from the external environment of the working chamber with clean air, introduce the clean air into the working chamber, and discharge the clean air toward the external environment via the hydrogen peroxide decomposition apparatus and the air outlet together with the hydrogen peroxide residual in the working chamber after decontamination,
the system being operated by the following 5 steps, wherein
in a step 1, the air inlet and the air outlet are closed, with the air circulating means operated, to maintain the working chamber in a sealed state and stop the air circulating means while the temperature and humidity in the working chamber are stable,
in a step 2, while the hydrogen peroxide solution supplying means is operated to supply a hydrogen peroxide solution from the storage tank to the mist converting/supplying means via the supply pump and the supply pipe, the weight and concentration of the hydrogen peroxide solution supplied to the mist converting/supplying means as a hydrogen peroxide solution are calculated over time from the amount and the concentration of a hydrogen peroxide solution supplied by the metering device and the concentration measuring device, and
while the mist converting/supplying means is operated to convert the total amount of the hydrogen peroxide solution supplied from the hydrogen peroxide solution supplying means into a mist for decontamination and supply the mist to the working chamber, the mist diffusion means is operated to diffuse the mist for decontamination to the working chamber,
in a step 3, at the end of supply of the mist for decontamination to the working chamber by the mist converting/supplying means, the mist converting/supplying means is stopped to maintain the mist diffusion means in an operated state for a predetermined decontamination duration,
in a step 4, at the end of the predetermined decontamination duration by the mist diffusion means, the mist diffusion means is stopped to open the air inlet and the air outlet, and operate the air circulating means and the aeration means, and
in a step 5, at the end of a predetermined aeration duration, the air circulating means and the aeration means are stopped and the air inlet and the air outlet are closed to maintain the working chamber in a sealed state,
wherein
based upon the temperature and humidity in the working chamber measured over time by the temperature measuring device and the humidity measuring device and the weight and concentration of a hydrogen peroxide solution supplied to the working chamber measured over time by the metering device and the concentration measuring device, the concentration of hydrogen peroxide gas in the working chamber is continuously calculated, using gas-liquid equilibrium of hydrogen peroxide - water.

2. The hydrogen peroxide decontamination system according to claim 1, wherein
in the step 3, the sealed state inside the working chamber is maintained, and the working chamber is decontaminated, with no material and/or heat flowing from the external environment into the working chamber.

3. The hydrogen peroxide decontamination system according to claim 1 or 2, wherein
the mist diffusion means includes one or more vibrating boards,
the vibrating board includes a base and a plurality of transmitters, and the plurality of transmitters is arranged on a plain surface of the base so as to be uniform in transmission directions, and the transmitters are operated in the same phase, and consequently
a sound flow is generated by a significantly directional ultrasound from the board surface of the vibrating board in the vertical direction by mutually amplifying ultrasounds of the plurality of transmitters in the front direction and mutually canceling out the ultrasounds of the plurality of transmitters in the lateral direction.
